# EUROPÄISCHE PATENTSCHRIFT

(11) **EP 0 387 752 B1**
(45) Veröffentlichungstag und Bekanntmachung des Hinweises auf die Patenterteilung: **12.01.1994**
(21) Anmeldenummer: 90104630.0
(22) Anmeldetag: 12.03.1990
(51) Int. Cl.: C12N 1/00, C12N 9/00, C12P 21/00

(54) **Verfahren zur Abreicherung von Salz aus Biomassesuspensionen**
Process for decreasing the salt of a biomass suspension
Procédé pour diminuer le sel d'une suspension biomasse

(30) Priorität: 15.03.1989 DE 3908421
(43) Veröffentlichungstag der Anmeldung: 19.09.1990
(73) Patentinhaber: FORSCHUNGSZENTRUM JÜLICH GMBH, 52425 Jülich (DE)
(72) Erfinder: Kula, Maria-Regina, Dr., D-5162 Niederzier (DE); Greve, Arnd, D-6237 Liederbach (DE)

(56) Entgegenhaltungen:
- BIOTECHNOLOGY ABSTRACTS, Derwent Publications LONDON, GB. Abstract n 91-03576A. GREVE ET AL: 'Recycling of salt from the primary bottom phase of a proteinextraction process - Salt recovery after protein purification by phasepartitioning with ethanol' &DECHEMA BIOTECHNOL. CONF. 1989 Vol. 3, Pt. B, p 1103-1106
- BIOTECHNOLOGY AND BIOENGINEERING. SYMP. 14 1984, NEW YORK US Seiten 573 - 579;T.L. DONALDSON: 'Phase separation of organics/water mixtures using salts'
- BIOTECHNOLOGY AND BIOENGINEERING. Bd. 33, Nr. 2, Februar 1989, NEW YORK USSeiten 221 - 228; R.N. PIKE ET AL.: 'Protein fractionation by three phasepartitioning (TPP) in aqueous/t-butanol mixtures'
- UCLA SYMP. MOL. CELL. BIOL., NEW SER. Bd. 68, 1987, NEW YORK, US Seiten 131 -148; R. LOVRIEN ET AL.: 'Three phase partitioning (TPP) via t-butanol: enzymesseparation from crudes'
- BIOCHIM. BIOPHYS. ACTA Bd. 221, Nr. 2, 17. November 1970, AMSTERDAM, NL Seiten387 - 390; G. JOHANSSON: 'Partition of salts and their effects on partition ofproteins in a dextran-poly(ethylene glycol)-water two-phase system'
- JOURNAL OF CHROMATOGRAPHY Bd. 216, 30. Oktober 1981, AMSTERDAM NL Seiten 346 -349; V. Y. RYASHENTSEV ET AL.: 'Behaviour of biomolecules in water-organicsolvent-inorganic salt two-phase ternary systems'
- ADV. BIOCHEM. ENG. Bd. 24, 1981, BERLIN Seiten 73 - 118; M-R. KULA ET AL.:'Purification of enzymes by liquid-liquid extraction'

## Beschreibung

Die Erfindung bezieht sich auf ein Verfahren zur Abreicherung von Salz aus Biomasse enthaltenden Suspensionen, insbesondere aus zellbruchstückhaltigen Flüssigkeiten der Proteingewinnung.

Bei der Gewinnung von intrazellulären Proteinen werden nach vorangehendem Aufschluß der Zellen wäßrige Phasensysteme aus Polyethylenglycol (PEG) und Salz (Phosphate, Sulfate oder Citrate) eingesetzt, um die Zellbruchstücke von den Proteinen zu trennen und die Proteine zu konzentrieren. Wäßrige Lösungen von Polymer und Salz (z.B. PEG mit einem Molekulargewicht von 1000 - 10000 und Kaliumphosphat) zerfallen oberhalb von Grenzkonzentrationen in wäßrige Phasen. In diesen Phasen lassen sich, je nach Konzentration der phasenbildenden Stoffe, Proteine, Zellen und Zellbruchstücke verschieden verteilen. So ist es möglich, durch geeignete Wahl des Phasensystems, die Zellbruchstücke vom gewünschten Protein abzutrennen und dieses Protein weiter zu reinigen (M.-R. Kula u.a., Adv. Biochem. Eng. 24 73- 118 bzw. DE-PS 26 39 129).

Dabei fällt als Abfall eine im allgemeinen zähflüssige Suspension mit Zellbruchstücken an, in der neben hochmolekularen Nukleinsäuren, löslichem und unlöslichem Protein, zwischen 10 und 25 % (w/w) Salz gelöst sind. Um sowohl die Verbrauchskosten zu senken, als auch die Umweltbelastung zu minimieren, sollte das Salz aus dieser biomassehaltigen Suspension zurückgewonnen werden.

Da die Zellbruchstücke einerseits eine Partikelgröße zwischen 0,05 und 5 µm (Untergrenze fließend) und einen geringen Dichteunterschied zur umgebenden Flüssigkeit haben und andererseits eine hohe Viskosität verursachen, ist das Salz mechanisch nur schwierig abzutrennen. Als bekannte Verfahren bieten sich die Mikrofiltration, Elektrodialyse oder die Wärmeagglomeration der Unterphase an, deren Durchführung jedoch nicht voll befriedigt.

Bei der Mikrofiltration müssen in Anbetracht der geringen Teilchengröße Membranen mit einer sehr geringen effektiven Trenngröße verwendet werden, bei denen selbst bei transmembranen Druckdifferenzen von 1 bar und Überströmgeschwindigkeiten von 5 m/s lediglich maximale Flüsse von ca. 25 l/m² h erzielt werden.

Bei der Elektrodialyse solcher Suspensionen sinkt die Grenzstromdichte infolge von Deckschichtbildungen aus Zellbruchstücken auf der Diluatseite der Membran erheblich ab, so daß ein kontinuierlicher Betrieb technisch nicht realisierbar ist.

Auch die Wärmeagglomeration und Abtrennung von Agglomerat stößt auf nicht unerhebliche Schwierigkeiten, insbesondere Trennprobleme, so daß der Einsatz dieses Verfahrens ebenfalls kaum zu erwarten ist.

Ziel der Erfindung ist daher ein Verfahren zur Abreicherung von Salz aus biomassehaltigen Suspensionen, insbesondere aus der salzhaltigen Unterphase einer wäßrigen 2-Phasen-Anreicherung intrazellulärer Proteine nach Zellaufschluß, das ohne Schwierigkeiten auch gewerblich angewandt werden kann.

Das zu diesem Zweck entwickelte erfindungsgemäße Verfahren ist dadurch gekennzeichnet, daß man das Salz aus der Suspension bzw. Flüssigkeit mit Alkohol unter Bildung einer salzhaltigen alkoholischen Oberphase extrahiert.

Wie gefunden wurde, entmischen Alkohol-Wasser-Salz-Systeme von wasserlöslichen Alkoholen bei bestimmter Zusammensetzung in zwei Phasen, wobei Zellbruchstücke in der Unterphase verbleiben. Das Salz verteilt sich zwischen Unterphase und Oberphase und kann in einer oder mehreren Stufe(n) aus der biomassehaltigen Unterphase zumindest soweit abgetrennt werden, daß deren Entsorgung keine Probleme bereitet.

Brauchbar ist dabei jeder Alkohol, der mit Wasser mischbar ist, aber selbst kein Lösungsvermögen für das Salz hat.

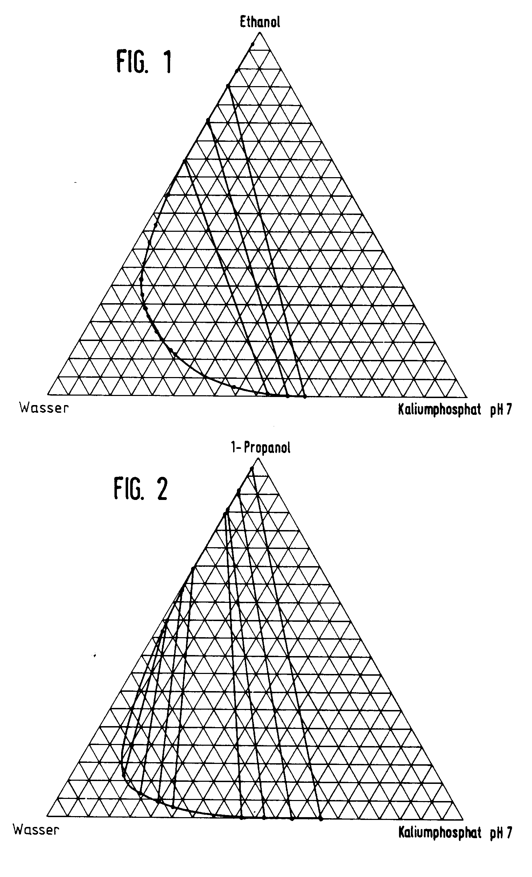

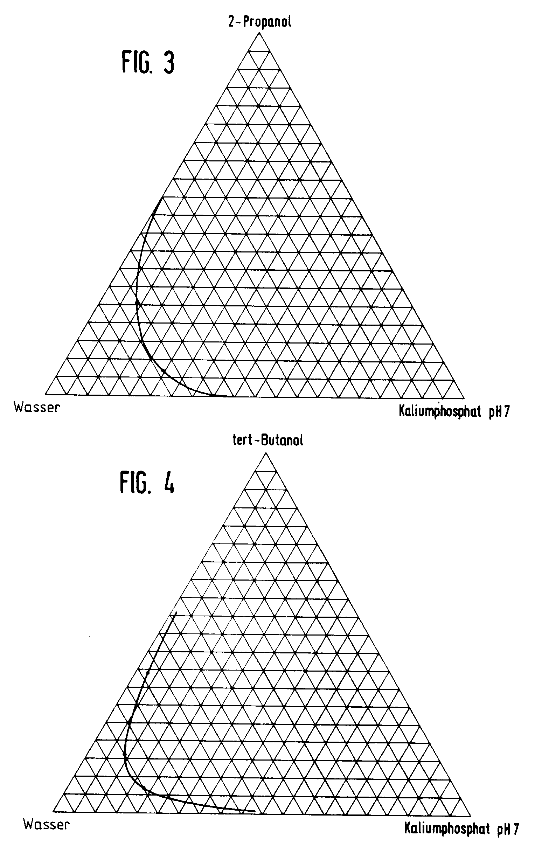

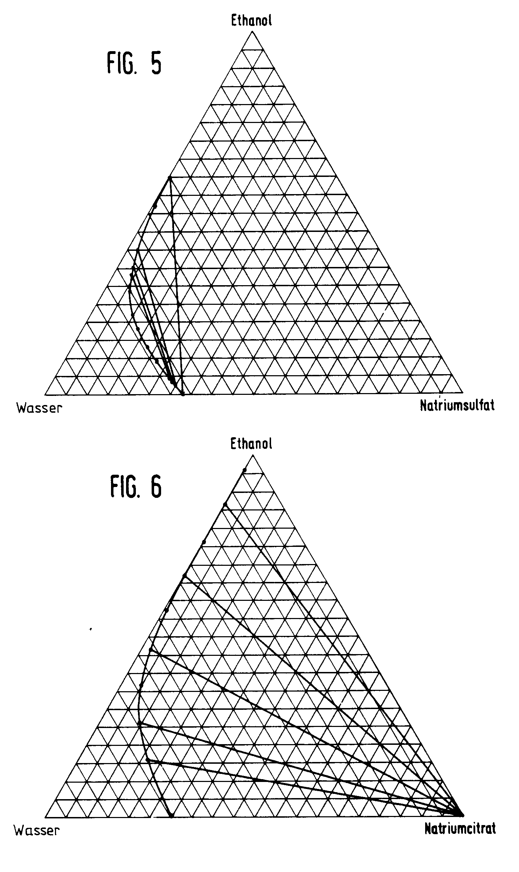

Besonders untersucht wurden die folgenden Alkohole:
- Methanol
- Ethanol
- 1-Propanol
- 2-Propanol
- tert-Butanol ,
für die - abgesehen von Methanol, dem wegen seines niedrigen Siedepunktes und der relativ hohen Konzentrationen für die Ausbildung von 2-Phasen-Systemen sowie der zugleich höchsten Verdampfungswärme innerhalb der Alkoholgruppe eine mindere praktische Bedeutung beigemessen wurde - Dreiecksdiagramme bei 25°C und unter Verwendung von Kaliumphosphat pH 7 (eine Mischung von KH₂PO₄ und K₂HPO₄) als das am meisten bei der Proteingewinnung verwendete Salz aufgenommen wurden (siehe Figuren 1 - 4). Die Nützlichkeit des Verfahrens für weitere Salze wurde am Beispiel Natriumsulfat und Natriumcitrat dargelegt (Figuren 5 und 6).

Da tert.-Butanol giftig ist und einen relativ hohen Schmelzpunkt (25,5°C) hat und Isopropanol teurer ist, wurden insbesondere 1-Propanol und Ethanol, insbesondere der letztere, umfänglicher untersucht.

Am zweckmäßigsten arbeitet man mit Mischungen, die im 2-Phasengebiet in der Nähe des kritischen Punktes liegen, wo die Zusammensetzungen von Ober- und Unterphase annähernd gleich sind, so daß eine maximale Abreicherung von Salz aus der biomassehaltigen Unterphase erreicht wird.

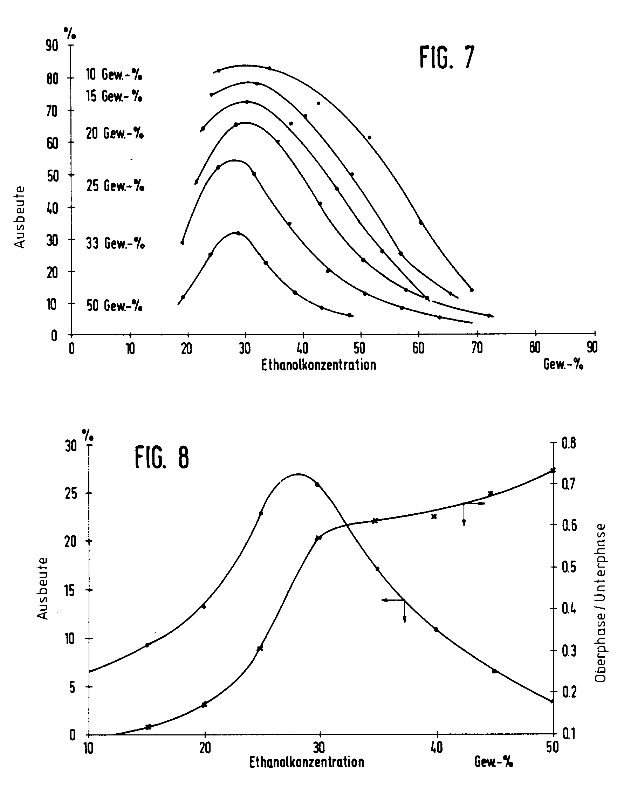

Der nützliche Arbeitsbereich des Verfahrens bei Verwendung von Ethanol ergibt sich für das biomassehaltige Modellsystem: 20 % (w/w) S. cerevisiae, 18 % (w/w) PEG 1500 und 7 % (w/w) Kaliumphosphat pH 7 aus den beigefügten Kurvenbildern (Figuren 7 und 8).

Für die praktische Extraktion des Salzes sind
- eine genügend große Dichtedifferenz zwischen den zu trennenden Phasen;
- die Trennung der Phasen in einzelnen Stufen einer mehrstufigen Extraktion in konstantem Volumenverhältnis sowie
- eine klare Oberphase
von Bedeutung, besonders wichtig ist jedoch die Ausbeute nach einer Stufe bzw. die Anzahl der theoretischen Stufen. Der zugesetzte Alkohol gelangt üblicherweise als wäßrige Lösung in das System.

Aus Figuren 7 und 8 läßt sich herleiten, daß eine 95%ige (w/w) Abtrennung von Kaliumphosphat in drei bis vier theoretischen Trennstufen möglich ist, wenn man im Gegenstrom arbeitet und mit Mischungsverhältnissen von 30 - 40 % (w/w) Ethanol im Gesamtsystem und einem Anteil der biomassehaltigen Suspension ("primäre Unterphase") von 15 - 25 % (w/w). Der Rest wird durch Wasser gebildet.

Figur 7 zeigt die Ausbeute (d.i. der Anteil Salz in der Oberphase bezogen auf die Gesamtmenge Salz nach einer Trennstufe) in Abhängigkeit von der Ethanolkonzentration (in Gew% der Mischung aus Ober-und Unterphase), wobei als Parameter die Anteile an biomassehaltiger Suspension in Gew% gewählt wurden. Die Kurvenschar zeigt, daß ein optimaler Übergang von Salz in die Oberphase erreicht wird, wenn der Suspensionsanteil möglichst gering ist. Mit abnehmendem Biomasseanteil nimmt jedoch die abtrennbare Salzmenge pro Trennstufe ab, so daß zweckmäßigerweise Suspensionsanteile von zumindest 15 Gew% eingesetzt werden.

Figur 8 zeigt den Einfluß der Alkoholkonzentration auf die Phasentrennung bei 50 Gew% Suspensionsanteil, welche durch das Massenverhältnis von Ober- zu Unterphase und die Ausbeute charakterisiert wird. Wie man sieht, wird ab etwa 30 Gew% Ethanolanteil der Gesamtmischung eine klare Phasentrennung mit annähernd gleichbleibendem Massenverhältnis von Ober- zu Unterphase erreicht.

Da die Ausbeute oberhalb von 30 Gew% Ethanol abnimmt, ist eine Arbeitsweise in Nähe dieser Konzentration bevorzugt.

Diese Verhältnisse werden durch die Art der Biomasse etwas beeinflußt: So werden für Salzextraktionen aus hefehaltigen Systemen etwas geringere Alkoholanteile verwendet als bei bakterienhaltigen Systemen. Generell sind daher Alkoholanteile von 30 - 50 % für Anteile an Zellsuspension zwischen 10 und 30 %, Rest Wasser besonders zweckmäßig.

Das erfindungsgemäße Verfahren wurde insbesondere im Zusammenhang mit der Gewinnung intrazellulärer Proteine durch Phasenverteilung entwickelt. Es ist jedoch selbstverständlich ebenso für andere Bereiche nützlich, bei denen Flüssigkeiten bzw. Suspensionen von Biomasse bzw. Proteinen anfallen, die mit hoher Salzfracht belastet sind.

Der Anteil der Zellsuspension wird dabei auf der einen Seite durch Kostenüberlegungen, auf der anderen Seite durch die Forderung einer ausreichenden Salzextraktion bestimmt: Wenn der Anteil der Zellsuspension zu gering ist, steigen die Kosten für das Verfahren, während bei einem zu hohen Anteil an Zellsuspension keine ausreichende Salzextraktion mehr möglich ist.

Die Alkoholanteile werden im wesentlichen durch den gewünschten Erfolg der Salzabtrennung bestimmt: So wird bei dem speziell untersuchten System mit Alkoholanteilen unter 30 % keine ausreichende Entmischung mehr erreicht, während mit Alkoholanteilen über 50 % zu wenig Wasser in der Oberphase ist, so daß die Salzabtrennung darunter leidet.

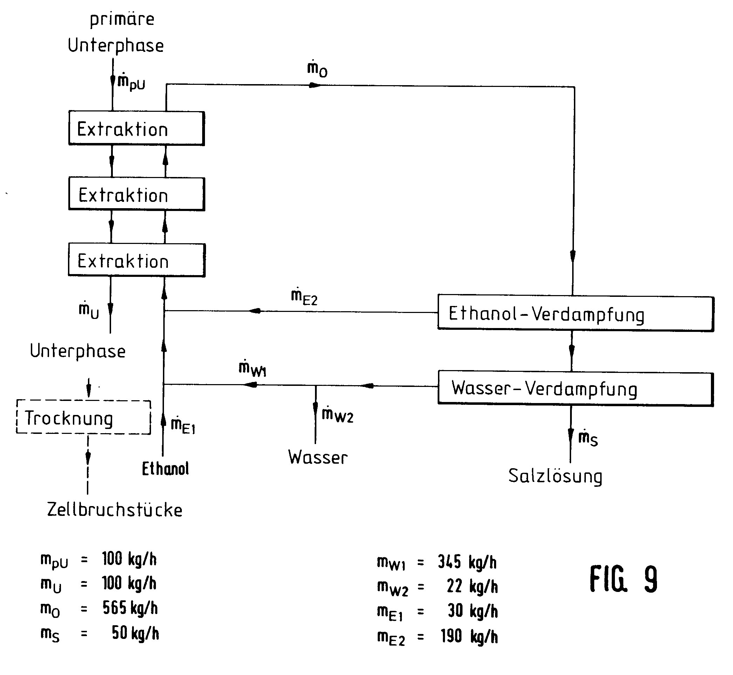

Die beigefügte Figur 9 zeigt ein Schema für die praktische Anwendung des erfindungsgemäßen Verfahrens der Salzabreicherung der biomassehaltigen Phase, wobei daneben (nach Abdampfung des Alkohols und von Wasser) eine Salzlösung erhalten wird, die zur Proteingewinnung rezykliert werden kann. Der Alkohol und ein Teil des abgedampften Wassers gelangen zur Salzextraktion zurück, in der primäre Unterphase (biomassehaltige Suspension) und wäßrige Alkohollösung im Gegenstrom durch drei Extraktionsstufen geführt werden.

## Patentansprüche

1. Verfahren zur Abreicherung von Salz aus Biomasse enthaltenden Suspensionen, insbesondere aus zellbruchstückhaltigen Flüssigkeiten der Proteingewinnung,
**dadurch gekennzeichnet,**
daß man das Salz aus der Suspension bzw. Flüssigkeit mit Alkohol unter Bildung einer salzhaltigen alkoholischen Oberphase extrahiert.

2. Verfahren nach Anspruch 1,
**dadurch gekennzeichnet,**
daß man als Alkohol Ethanol, Propanol, Isopropanol oder tert.-Butanol, insbesondere Ethanol verwendet.

3. Verfahren nach Anspruch 1 oder 2,
**dadurch gekennzeichnet,**
daß als Suspension bzw. Flüssigkeit eine Zellbruchstücke, lösliches und unlösliches Protein sowie Nukleinsäure und Salz enthaltende Unterphase einer wäßrigen Zweiphasenextraktion von intrazellulären Proteinen, insbesondere Enzymen, verarbeitet wird.

4. Verfahren nach einem der vorangehenden Ansprüche,
**dadurch gekennzeichnet,**
daß man für die Extraktion 10 bis 30 Gew% salzhaltige Suspension bzw. Flüssigkeit mit 30 bis 50 Gew% Alkohol, insbesondere Ethanol, Rest Wasser verwendet.

5. Verfahren nach Anspruch 4,
**dadurch gekennzeichnet,**
daß die Extraktion in mehreren aufeinanderfolgenden Stufen, insbesondere als Gegenstromextraktion in mindestens drei Stufen durchgeführt wird.

6. Verfahren nach einem der vorangehenden Ansprüche,
**dadurch gekennzeichnet,**
daß die Trennung der Oberphase von der Unterphase durch Tellerseparatoren oder Dekanter vorgenommen wird.

7. Verfahren nach einem der vorangehenden Ansprüche,
**dadurch gekennzeichnet,**
daß der Alkohol aus der anfallenden salzreichen Oberphase abgedampft und die Salzlösung ggf. nach weiterer Aufkonzentrierung zur Proteingewinnung rezykliert wird.

## Claims

1. A method for the depletion of salt from suspensions containing biomass, particularly from liquids from protein production and containing cell fragments, characterised in that the salt is extracted from the suspension or liquid using alcohol with the formation of a salt-containing alcoholic upper phase.

2. A method according to claim 1, characterised in that ethanol, propanol, isopropanol or tert.-butanol, particularly ethanol, is used as the alcohol.

3. A method according to claims 1 or 2, characterised in that a lower phase from an aqueous two-phase extraction of intracellular proteins, particularly enzymes, and containing cell fragments, soluble and insoluble protein, and nucleic acid and salt, is worked up as the suspension or liquid.

4. A method according to any one of the preceding claims, characterised in that 10 to 30 weight % suspension or liquid containing salt, with 30 to 50 weight % alcohol particularly ethanol, balance water, is used for the extraction.

5. A method according to claim 4, characterised in that the extraction is performed in a plurality of successive stages, particularly as a counter-current extraction in at least three stages.

6. A method according to any one of the preceding claims, characterised in that the separation of the upper phase from the lower phase is effected by means of disc separators or decanters.

7. A method according to any one of the preceding claims, characterised in that the alcohol is evaporated off from the salt-rich upper phase arising and the salt solution is recycled, optionally after further concentration, for the production of protein.

## Revendications

1. Procédé d'appauvrissement du sel dans des suspensions contenant de la biomasse, en particulier des liquides contenant des fragments cellulaires, provenant de l'obtention de protéines, caractérisé en ce qu'on extrait le sel de la suspension ou du liquide avec un alcool, avec formation d'une phase supérieure alcoolique contenant du sel.

2. Procédé selon la revendication 1, caractérisé en ce qu'on utilise, comme alcool, de l'éthanol, du propanol, de l'isopropanol ou du tert.-butanol, en particulier de l'éthanol.

3. Procédé selon la revendication 1 ou 2, caractérisé en ce qu'on traite, comme suspension ou liquide, une phase inférieure, contenant des fragments cellulaires, de la protéine soluble et de la protéine insoluble, ainsi que de l'acide nucléique et du sel, provenant d'une extraction aqueuse à deux phases de protéines intracellulaires, en particulier des enzymes.

4. Procédé selon l'une des revendications précédentes, caractérisé en ce qu'on utilise, pour l'extraction, de 10 à 30 % en poids d'une suspension ou d'un liquide contenant du sel, avec 30 à 50 % en poids d'alcool, en particulier de l'éthanol, le reste étant de l'eau.

5. Procédé selon la revendication 4, caractérisé en ce qu'on effectue l'extraction dans plusieurs étapes successives, en particulier sous forme d'extraction à contre-courant dans au moins trois étapes.

6. Procédé selon l'une des revendications précédentes, caractérisé en ce qu'on effectue la séparation de la phase supérieure et de la phase inférieure grâce à des séparateurs à plateau ou des décanteurs.

7. Procédé selon l'une des revendications précédentes, caractérisé en ce que l'alcool de la phase supérieure saline obtenue est évaporé, et la solution saline est recyclée, éventuellement après une concentration complémentaire, pour l'obtention de protéines.
